# EUROPEAN PATENT APPLICATION

(11) **EP 1 550 726 A1**
(43) Date of publication of application: **06.07.2005**
(21) Application number: 02777832.3
(22) Date of filing: 11.10.2002
(51) Int. Cl.: C12P 17/08, C07D 313/00, A61K 31/365, A61P 31/10, A61P 31/04

(54) **NOVEL K99-5278 SUBSTANCES AND PROCESS FOR PRODUCING THE SAM E**

(71) Applicant: THE KITASATO INSTITUTE, Tokyo 108-8642 (JP)
(72) Inventor: OMURA, Satoshi, Setagaya-ku, Tokyo 157-0076 (JP); TOMODA, Hiroshi, Chofu-shi, Tokyo 182-0034 (JP); TAKAHASHI, Yoko, Suginami-ku, Tokyo 168-0073 (JP)
(74) Representative: Duckworth, Timothy John
(86) International application number: PCT/JP2002/010586
(87) International publication number: WO 2004/033702

(57) **Abstract**

The present invention relates to effective drugs for mycosis caused by so called fungi such as fungi and yeast. The present invention is comprised of culturing a microorganism belonging to genus Streptomyces and having ability to produce K99-5278A substance, K99-5278B substance and K99-5278C substance in a medium, accumulating K99-5278A substance, K99-5278B substance and K99-5278C substance in the cultured medium and isolating K99-5278A substance, K99-5278B substance and K99-5278C substance from the cultured mass. The thus obtained substances can be expected as a novel antifungal agent.

## Description

### Technical Field

The present invention relates to novel K99-5278 substance having antifungal activities against fungi and yeasts and a process for production thereof. In the present invention, K99-5278 substance includes K99-5278A substance, K99-5278B substance and K99-5278C substance.

### Background Art

Hitherto known numbers of chemotherapeutic agents against mycosis caused by fungi and yeasts are extremely limited. More particularly, practically used drugs at present are azoles used for treatment of disease such as deep-seated mycosis, for example:
1-[(2,4-dichlorobenzyloxy)-2-(2,4-dichlorophenyl)ethyl imidazole (generic name: miconazole, Sigma Inc.);
2,4-difluoro-α,α-bis(1H -1,2,4-triazol-1-ylmethyl)benzyl alcohol (generic name: fluconazole, I.C.N. Pharmaceuticals Inc.); and
(±)-1-Sec-butyl-4-[P-[2R,4S]-2-(2,4-dichlorophenyl)-2-(1H-1 ,2,4-triazol-1-ylmethyl)1,3-dioxolan-4-yl]methoxy]phenyl]-1 -piperazinyl]phenyl]-Δ²-1,2,4-triazolin-5-one (generic name: itraconazole, Kyowa Hakko Kogyo Co.), which inhibit C-14 demethylation of ergosterol, amphotericin B which is a polyene cell membrane inhibitor and flucytosine which is a DNA synthesis inhibitor.

Miconazole, fluconazole and itraconazole were reported to be highly safety as compared with polyenes and were used most frequently (Anaisssie E. J. et al. Clin. Infect. Diseases, 23, 964-972, 1966).

However, since long-term or repeated administration of these antimicrobial agents resulted to appear resistant microbes, novel antifungal agents having different chemical structures or different mechanism of actions are highly desired.

### Disclosure of the invention

In such circumstances, if novel antifungal agents having new chemical structure useful for fungal infectious disease caused by infection of fungi or yeasts, it can be useful chemotherapeutic agents against mycosis.

An aspect of the present invention is to provide novel K99-5278 substance, which can be satisfied for such the expectation, and a process for production thereof.

In order to solve such the problems hereinabove described, we have continued studies on microbial metabolites, and found that substances having antifungal activities were produced in the cultured medium of a newly isolated strain, designated as K99-5278, from soil sample. Further, we have found that substances represented by the chemical structure of the formula [I], [II] and [III] hereinbelow as a result of isolation and purification of the active principle showing antifungal activities from the cultured mass. Since substances having such chemical structures were not known, these substances were designated as K99-5278A substance, K99-5278B substance and K99-5278C substance, and were totally designated as K99-5278 substance. The present invention has completed by such knowledge.

An object of the present invention is to provide K99-5278A substance represented by the following formula [I]; 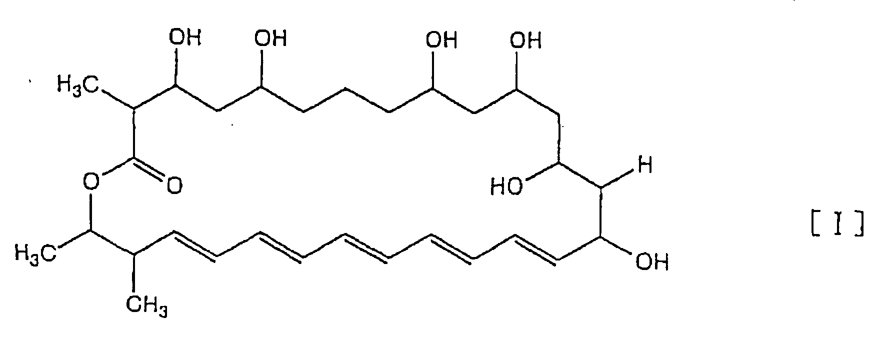 K99-5278B substance represented by the following formula [II]; 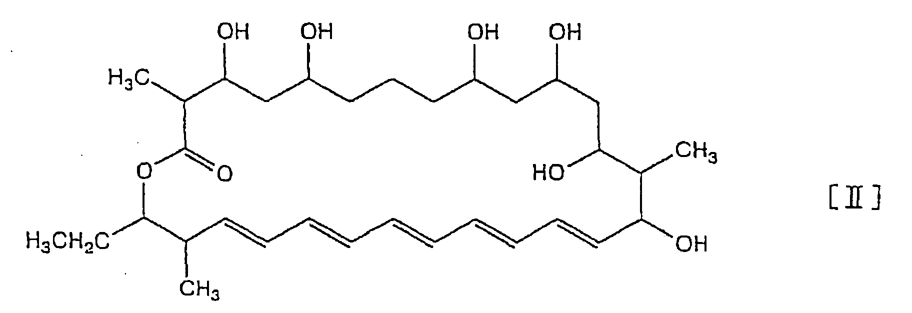 and K99-5278C substance represented by the following formula [III]

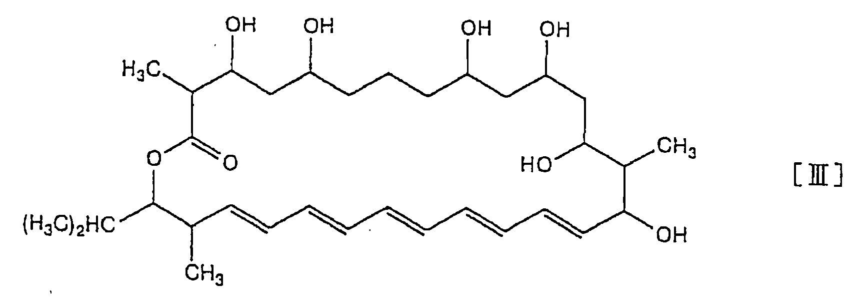

Another object of the present invention is to provide a process for production of novel K99-5278 substance comprising culturing microorganism having ability to produce K99-5278 substance, accumulating K99-5278 substance in a cultured medium and isolating K99-5278 substance from the cultured mass.

Further object of the present invention is to provide a process for production of novel K99-5278 substance wherein a microorganism belonging to genus Streptomyces and having ability to produce K99-05278 substance is Streptomyces sp. K99-5278 FERM BP-8198.

Still further object of the present invention is to provide a microorganism belonging to genus Streptomyces and having ability to produce K99-05278 substance.

Still another object of the present invention is to provide a microorganism wherein the microorganism belonging to genus Streptomyces and having ability to produce K99-05278 substance is Streptomyces sp. K99-5278 FERM BP-8198.

Another object of the present invention is to provide K99-5278 substance wherein K99-5278A substance has antimicrobial activities against Aspergillus niger KF103, Mucor racemosus KF223, Candida albicans KFI and Saccharomyces cerevisiae KF26.

Another object of the present invention is to provide K99-5278 substance wherein K99-5278B substance has antimicrobial activities against Aspergillus niger KF103, Mucor racemosus KF223, Candida albicans KFI and Saccharomyces cerevisiae KF26.

Another object of the present invention is to provide K99-5278 substance wherein K99-5278C substance has antimicrobial activities against Aspergillus niger KF103, Mucor racemosus KF223, Candida albicans KFI and Saccharomyces cerevisiae KF26.

The microorganism having ability to produce K99-5278 substance represented by the formula [I], [II] and [III] hereinbefore (hereinafter designates as "K99-5278 substance producing microorganism") belongs to genus Streptomyces, and, for example, Streptomyces sp. K99-5278, which was newly isolated from soil sample collected in Minato-ku, Tokyo by us, is the most preferable strain.

Taxonomical properties of Streptomyces sp. K99-5278 of the present invention are as follows.

### (I) Morphological properties

Vegetative mycelia grow well on various agar media and no fragmentation is observed. Aerial mycelia are abundantly grown on yeast extract-malt extract agar medium and oatmeal agar medium, and exhibit white to dark brownish color. On microscopic observation, chains of more than 20 spores are observed on the aerial mycelia, and the morphological form is linear chains and size of spore is about 1.2 × 0.8 µm with cylindrical form. Surface of the spore is smooth. Sclerotia, sporangia and flagellate spore are not observed.

### (II) Culture properties on various media

Culture properties of the producing strain of the present invention determined by the method of E. B Shirling and D. Gottlieb (International Journal of Systematic Bacteriology, 16: 313, 1966) are shown in the following. Color tone was determined referring to Color Harmony Manual, 4th Ed. (Container Corporation of America, Chicago, 1958) as a standard color, and color name as well as attached code number in the parenthesis. Unless otherwise noted, results are observation of cultures at 27°C for 2 weeks on various media.

| Sucrose-nitrate agar medium | |
|---|---|
| Growth | moderate, alabaster tint (13ba) |
| Reverse side | pearl (2ba) |
| Aerial mycelium | moderate, white (a) |
| Soluble pigment | none |

| Glucose-asparagine agar medium | |
|---|---|
| Growth | moderate, biscuit (2ec) |
| Reverse side | biscuit (2ec) |
| Aerial mycelium | none |
| Soluble pigment | none |

| Glycerol-asparagine agar medium (ISP) | |
|---|---|
| Growth | good, biscuit (2ec) |
| Reverse side | biscuit (2ec) |
| Aerial mycelium | moderate, orchid tint (10ba) |
| Soluble pigment | yellow |

| Inorganic salts-starch agar medium (ISP) | |
|---|---|
| Growth | good, light ivory (2ca) |
| Reverse side | light wheat (2ea) |
| Aerial mycelium | moderate, white (a) |
| Soluble pigment | none |

| Tyrosine agar medium (ISP) | |
|---|---|
| Growth | poor, ivory (2db) |
| Reverse side | ivory (2db) |
| Aerial mycelium | poor, white (a) |
| Soluble pigment | none |

| Oatmeal agar medium (ISP) | |
|---|---|
| Growth | good, camel (3ie) |
| Reverse side | fawn (4ig) |
| Aerial mycelium | abundant, rosewood (5ge) |
| Soluble pigment | none |

| Yeast extract-malt extract agar medium (ISP) | |
|---|---|
| Growth | good, chestnut brown (4ni) |
| Reverse side | light spice brown (4lg) |
| Aerial mycelium | abundant, sand (3cb)-no name (5cb) |
| Soluble pigment | none |

| Nutrient agar medium (ISP) | |
|---|---|
| Growth | moderate, bisque (3ec) |
| Reverse side | light wheat (2ea) |
| Aerial mycelium | none |
| Soluble pigment | yellow |

| Peptone-yeast extract-iron agar medium (ISP) | |
|---|---|
| Growth | poor, light mustard tan (2ie) |
| Reverse side | mustard (21e) |
| Aerial mycelium | none |
| Soluble pigment | yellow |

| Glucose-nitrate agar medium | |
|---|---|
| Growth | poor, pearl (3ba) |
| Reverse side | pearl (3ba) |
| Aerial mycelium | none |
| Soluble pigment | none |

| Glycerol-calcium malate agar medium | |
|---|---|
| Growth | good, bisque (3ec) - camel (3ie) |
| Reverse side | bisque (3ec) - camel (3ie) |
| Aerial mycelium | scant, white (a) |
| Soluble pigment | none |

| Glucose-peptone agar medium | |
|---|---|
| Growth | moderate, camel (3ie) |
| Reverse side | light amber (3ic) |
| Aerial mycelium | moderate, white (a) |
| Soluble pigment | yellow |

### (III) Physiological properties

| | | |
|---|---|---|
| (1) | Formation of melanin pigment | |
| | (a)Tyrosine agar | negative |
| | (b)Peptone-yeast extract-iron agar medium | negative |
| | (c)Tryptone-yeast liquid | negative |
| | (d)Simple gelatin medium (21 - 23°C) | negative |
| (2) | Nitrate reduction | positive |
| (3) | Liquefaction of gelatin (21 - 23°C) (gelatin medium) | negative |
| (4) | Starch hydrolysis | positive |
| (5) | Coagulation of defatted milk (27°C) | negative |
| (6) | Peptonization of defatted milk (27°C) | positive |
| (7) | Growth temperature | 19 - 35°C |
| (8) | Utilization of carbon sources (Pridham-Gottlieb agar medium) | |
| | Utilize: D-glucose, melibiose myo-inositol | |
| | Slightly utilize: L-arabinose, raffinose | |
| | Not utilize: D-xylose, D-mannitol, D-fructose, L-rhamnose, myo-inositol, sucrose | |
| (9) | Decomposition of cellulose | negative |

### (IV) Composition of cell wall

2,6-diaminopimelic acid of cell wall is LL type. Main menaquinones are MK-9 (H₆) and MK-9 (H₈).

### (V) Conclusion

Taxonomical properties of the strain of the present invention are summarized as follows. 2,6-diaminopimelic acid in the cell wall is LL type and main menaquinones are MK-9 (H₆) and MK-9 (H₈). Morphology of the spore chain is straight chain, forming with long spore chains and smooth spore surface. Various properties on the culture are exhibiting pale yellow to brown color tone vegetative mycelia and white to grayish aerial mycelia. No production of melanin pigment is observed, but yellow soluble pigments formation was observed.

The present strain exhibiting the above morphological properties, culture properties and physiological properties was identified as the strain belonging to genus Streptomyces, by Williams et al. (Bergey's Manual of Determinative Bacteriology, 8th Ed., page 748-2492, 1974). The strain was deposited as Streptomyces sp. K99-5278 in International Patent Organism Depository, National Institute of Advanced Industrial Science and Technology, AIST Tsukuba Central 6, 1-1, Higashi 1-chome, Tsukuba-shi, Ibaraki-ken, 305-8566 Japan on September 27, 2002 as permanent depository number FERM BP-8198.

The strain K99-5278 can be mentioned as a preferable example of K99-5041 substance producing strain used in the present invention. However, since the morphological properties of microorganisms are generally very easily mutated and are not constant. Natural mutation or artificial mutation generally performed by ultraviolet irradiation or chemical mutagens such as N-methyl-N'-nitro-N-nitrosoguanidine and ethyl methansulfonate, are well known. The strain belonging to genus Streptomyces and having ability to produce K99-5278 substance represented by the formula [I], [II] and [III] hereinbefore, including the artificial mutants as well as natural mutants, can be used in the present invention.

In production of K99-5278 substance of the present invention, at first, K99-5278 substance producing strain belonging to genus Streptomyces is cultured in a preferable medium. Nutrient sources preferable for production of K99-5278 substance of the present invention are assimilable carbon sources for microorganism, digestible nitrogen sources and, if necessary, inorganic salts. Examples of assimilable carbon sources are sugars such as glucose, maltose, lactose, galactose, dextrin and starch, and plant oil such as soybean oil, etc. are used independently or in combination.

Examples of nitrogen sources are peptone, yeast extract, meat extract, soybean powder, cotton seed powder, corn steep liquor, malt extract, casein, amino acids, urea, ammonium salts and nitrates are used independently or in combination. If necessary, salts such as phosphate, magnesium, calcium, sodium, potassium, heavy metallic salts such as iron, manganese, copper, cobalt or zinc, vitamins and substances suitable for production of K99-5278 substance are added.

In the liquid culture, if foaming occurs, antifoam agents such as liquid paraffin, animal oil, vegetable oil, silicone oil and surface active agent can preferably be added. The above culture can be performed by liquid or solid culture condition, if the above nutrient sources are contained, and in general, the culture can preferably be performed using liquid culture medium, and in case of small production, the culture using flask is preferable.

In the large scale production using the large tank, in order to prevent delay of growth of microorganism in the production process, the production strain is inoculated and cultured initially in relatively small amount of culture medium, subsequently the cultured mass is transferred into the large tank and cultivation is preferably continued. In this case, compositions of the medium used in the pre-culture and the medium used in the production culture can be identical or different if necessary.

In the culture under aeration spinning condition, conventional means, for example, agitation using propeller and other mechanical stirring, rotation or shaking in fermenter, treating with pumping and blowing air can be applied. Air for aeration should be sterilized. Culturing temperature can be applied within ranges in the production of K99-5278 substance by K99-5278 substance producing strain, and the cultivation is performed usually at 19 - 35°C, preferably at 27°C. Culturing pH is usually pH 6 - 8, preferably about pH 7. Culturing time depends on culturing condition and is usually for 7 days.

The thus obtained accumulated K99-5278 substance in the cultured mass exists generally in cultured mycelia. Isolatio n of K99-5278 substance from the cultured mycelia can be perf ormed by methods used for isolation of metabolites from micro bial cultured mass independently, repeatedly or in combinatio n with any orders of the means. For example, means such as fi ltration, centrifugation, dialysis, concentration, drying, f reeze drying, adsorption, desorption, a method for applying d ifference in solubility for various solvents (e.g. precipitat ion, crystallization, recrystallization, transfer, counter c urrent distribution, etc.) and chromatography can be applied.

Isolation and collection of K99-5278 substance can be performed by collecting from the mycelial extract, for example, extracting the whole cultured mass with organic solvent such as acetone, ethanol or methanol. The extract is concentrated and is extracted with organic solvent such as chloroform and ethyl acetate. After concentration of the extract, K99-5278A substance, K99-5278B substance and K99-5278C substance can be isolated by chromatography such as silica gel column chromatography, Sepahdex LH-20 column chromatography and ODS column chromatography.

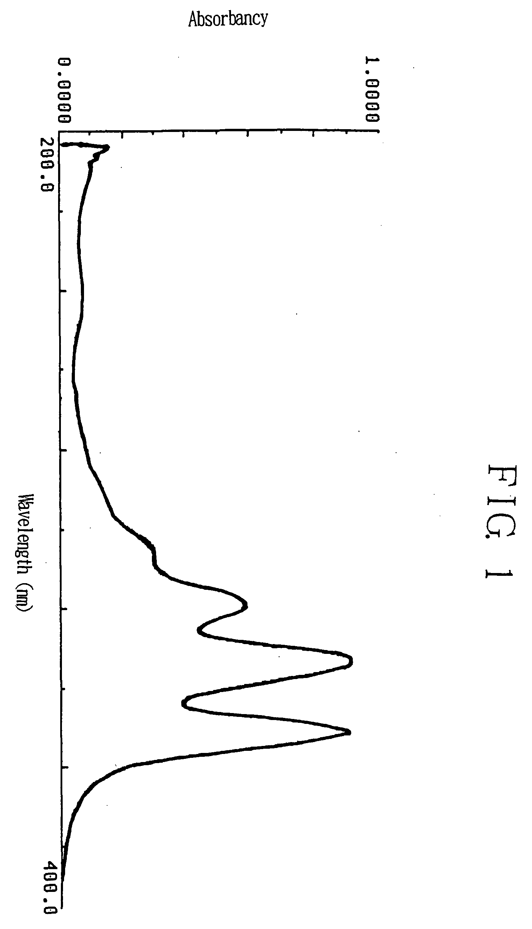

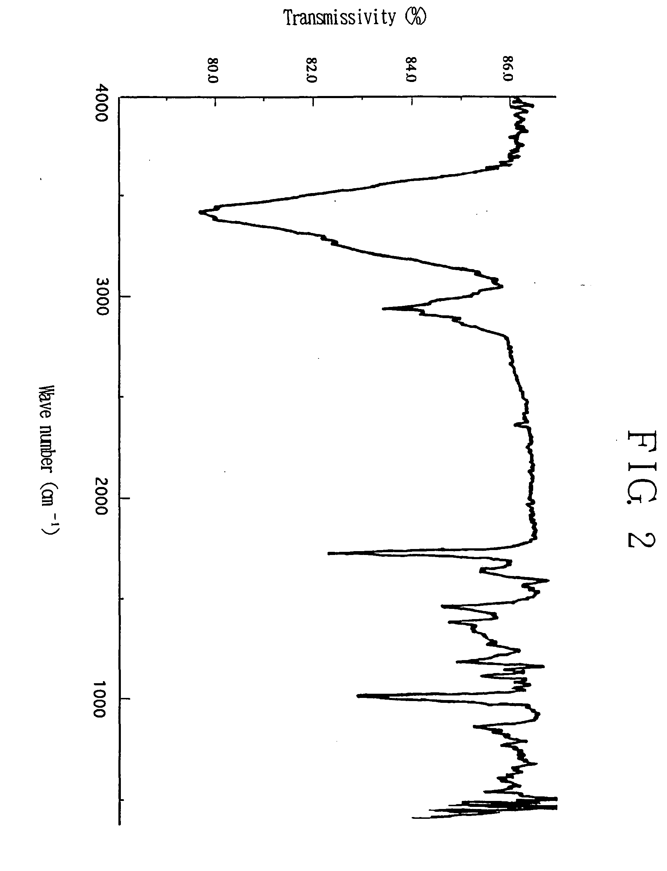

Physicochemical properties of K99-5278 substance of the present invention are explained hereinbelow.
[I] K99-5278A substance
(1) Nature: pale yellow powder
(2) Melting point: 160°C
(3) Molecular formula: C₃₀H₄₈O₈ HRFAB-MS(m/z) [M+Na]
Calculated 559.3246, Found 559.3247
(4) Molecular weight: 536
(5) Ultraviolet absorption spectrum (in methanol): as shown in Fig. 1, specific absorption maximum λmax at 306 nm, 319 nm, 334 nm and 352 nm
(6) Infrared absorption spectrum (KBr Tablet) : as shown in Fig. 2, specific maximum absorption λmax at 3415, 2937, 1726, 1010 cm⁻¹.
(7) Specific rotation: [α]_{D}²³= -15.9° (c= 0.1, methanol)
(8) Solubility in solvent: soluble in dimethyl sulfoxide (DMSO) and methanol, slightly soluble in ethyl acetate and chloroform, and insoluble in hexane and water.
(9) Grouping for acidic, neutral and basic: Neutral substance.
(10) ¹H-proton nuclear magnetic resonance spectrum (in deuteriochloroform) measured by using Varian NMR 400 MHz: Chemical shift of hydrogen (ppm) is shown in Table 1.
(11) ¹³C-nuclear magnetic resonance spectrum (in deuteriochloroform) measured by using Varian NMR 100 MHz: Chemical shift of carbon (ppm) is shown in Table 1.

**Table 1**

| ¹³C | ¹H |
|---|---|
| 173.9 | |
| 45.9 | 2.24 (1H, m) |
| 71.8 | 3.75 (1H, br, m) |
| 41.6 | 1.20 (2H, m) |
| 70.8 | 3.33 (1H, br, m) |
| 38.2 | 1.20 (2H, m) |
| 22.3 | 1.60 (2H, m) |
| 38.8 | 1.20 (2H, m) |
| 71.0 | 3.49 (1H, br, m) |
| 43.7 | 1.20 (2H, m) |
| 68.8 | 3.75 (1H, br, m) |
| 43.9 | 1.20 (2H, m) |
| 65.4 | 3.33 (1H, m) |
| 44.2 | 1.60 (2H, m) |
| 68.6 | 4.08 (1H, m) |
| 137.7 | 5.57 (1H, dd, J=15.20, 7.69) |
| 129.7 | 6.10 (1H, m) |
| 133-131.3 | 6.23 (1H, m) |
| 133-131.3 | 6.23 (1H, m) |
| 133-131.3 | 6.23 (1H, m) |
| 133-131.3 | 6.23 (1H, m) |
| 133-131.3 | 6.23 (1H, m) |
| 133-131.3 | 6.23 (1H, m) |
| 129.7 | 6.10 (1H, m) |
| 136.3 | 5.82 (1H, dd, J=15.20, 6.78) |
| 40.4 | 2.32 (1H, m) |
| 73.4 | 4.62 (1H, m) |
| 12.6 | 1.02 (3H, d, J=6.96) |
| 15.9 | 0.98 (3H, d, J=6.95) |
| 18.8 | 1.45 (H, d, J=6.23) |

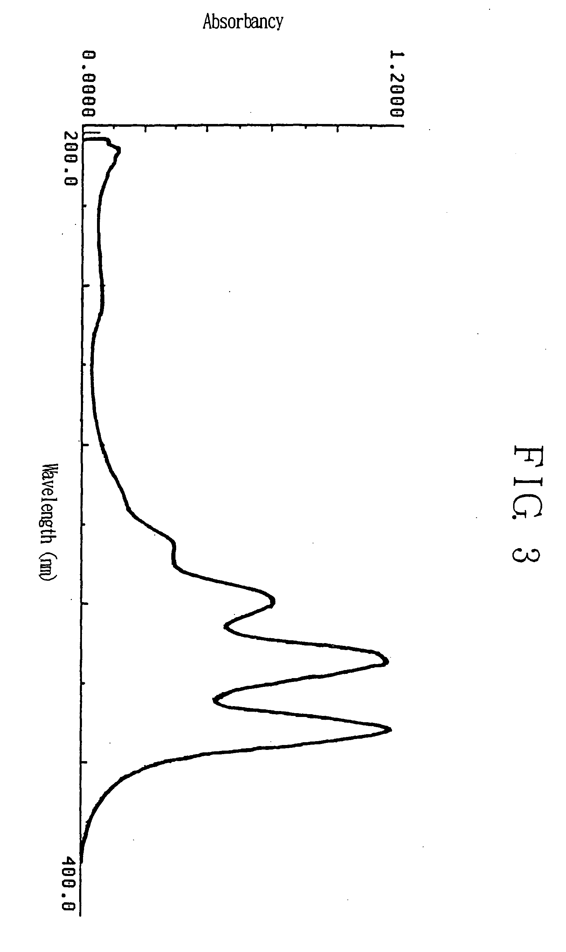

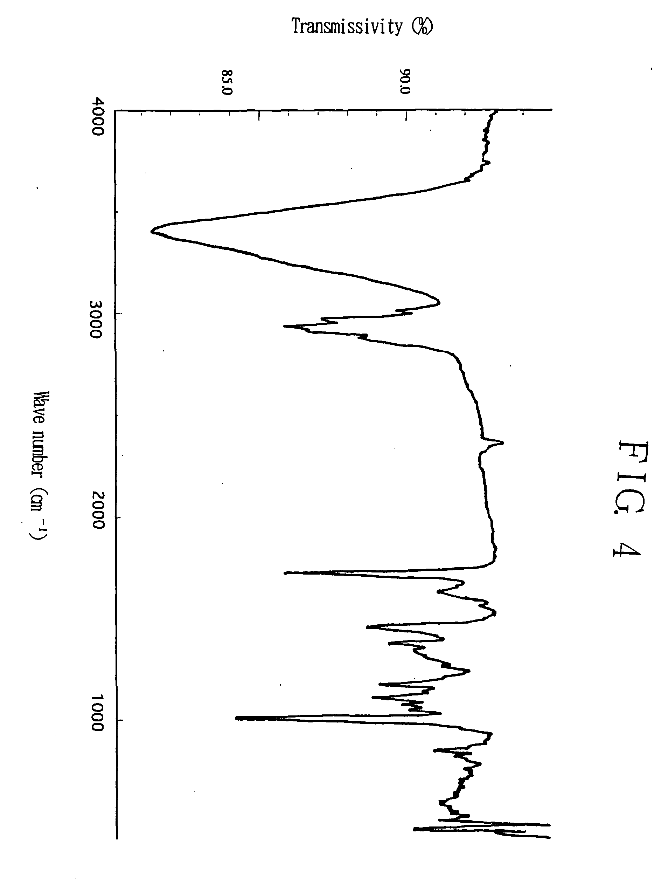

As shown in above, as a result of detailed examination of various physico-chemical properties and spectral data of K99-5278A substance, K99-5278A substance was determined to have the chemical structure as shown in the formula [I].
[II] K99-5278B substance
(1) Nature: pale yellow powder
(2) Melting point: 155°C
(3) Molecular formula: C₃₂H₅₂O₈ HRFAB-MS(m/z) [M+Na]
Calculated 587.3559, Found 587.3561
(4) Molecular weight: 564
(5) Ultraviolet absorption spectrum (in methanol): as shown in Fig. 3, specific absorption maximum λmax at 306 nm, 319 nm, 335 nm and 352 nm
(6) Infrared absorption spectrum (KBr Tablet) : as shown in Fig. 4, specific maximum absorption λmax at 3405, 2937, 1726, 1006 cm⁻¹.
(7) Specific rotation: [α]_{D}²³= -63.9° (c= 0.1, methanol)
(8) Solubility in solvent: soluble in dimethyl sulfoxide (DMSO) and methanol, and insoluble in hexane, ethyl acetate, chloroform and water.
(9) Grouping for acidic, neutral and basic: Neutral substance.
(10) ¹H-proton nuclear magnetic resonance spectrum (in deuteriochloroform) measured by using Varian NMR 400 MHz: Chemical shift of hydrogen (ppm) is shown in Table 2.
(11) ¹³C-nuclear magnetic resonance spectrum (in deuteriochloroform) measured by using Varian NMR 100 MHz: Chemical shift of carbon (ppm) is shown in Table 2.

**Table 2**

| ¹³C | ¹H |
|---|---|
| 174.2 | |
| 46.0 | 2.28 (1H, m) |
| 71.9 | 3.71 (1H, br, m) |
| 41.6 | 1.20 (2H, m) |
| 71.4 | 3.40 (1H , br, m) |
| 38.8 | 1.20 (2H, m) |
| 22.5 | 1.70 (2H, m) |
| 40.1 | 1.20 (2H, m) |
| 71.4 | 3.58 (1H, br, m) |
| 43.5 | 1.20 (2H, m) |
| 69.5 | 3.78 (1H, br, m) |
| 38.3 | 1.20 (2H, m) |
| 67.6 | 3.40 (1H, m) |
| 45.4 | 1.45 (2H, m) |
| 74.5 | 3.58 (1H, m) |
| 136.7 | 5.54 (1H, dd, J=15.02, 8.61) |
| 130.4 | 6.06 (1H, m) |
| 133-131.2 | 6.21 (1H, m) |
| 133-131.2 | 6.21 (1H, m) |
| 133-131.2 | 6.21 (1H, m) |
| 133-131.2 | 6.21 (1H, m) |
| 133-131.2 | 6.21 (1H, m) |
| 133-131.2 | 6.21 (1H, m) |
| 129.6 | 6.06 (1H, m) |
| 136.1 | 5.83 (1H, dd, J=15.20, 5.83) |
| 38.0 | 2.38 (1H, m) |
| 77.7 | 4.59 (1H, td, J=8.42, 3.30) |
| 13.1 | 1.07 (3H, d, J=7.47) |
| 10.5 | 0.85 (3H, d, J=6.95) |
| 15.8 | 0.99 (3H, d, J=6.77) |
| 25.2 | 1.60 (2H, m) |

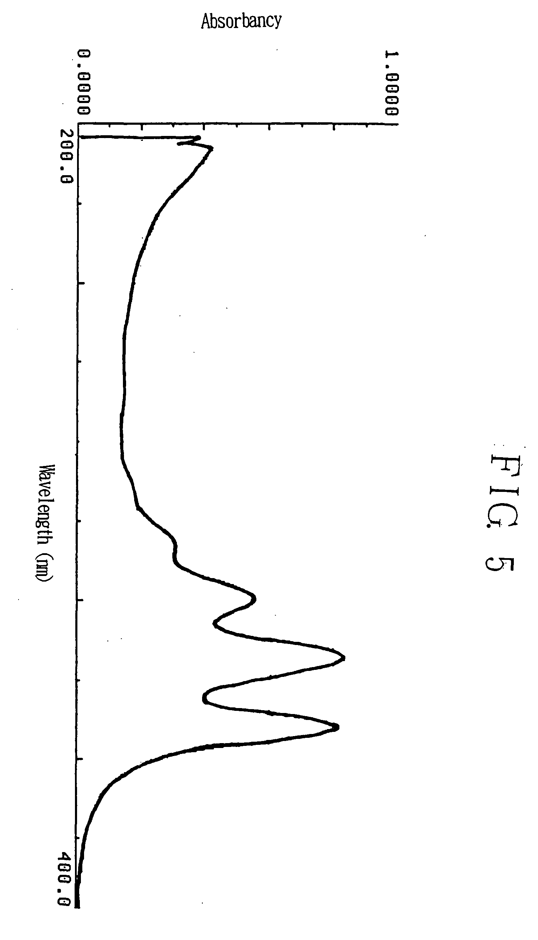

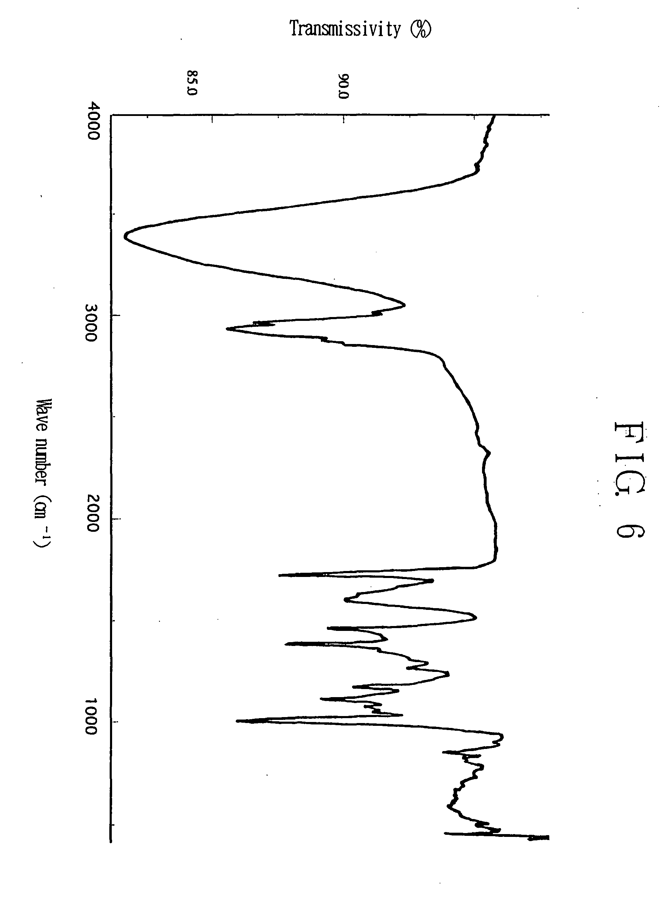

As shown in above, as a result of detailed examination of various physico-chemical properties and spectral data of K99-5278B substance, K99-5278B substance was determined to have the chemical structure as shown in the formula [II].
[III] K99-5278C substance
(1) Nature: pale yellow powder
(2) Melting point: 156°C
(3) Molecular formula: C₃₃H₅₄O₈ HRFAB-MS (m/z) [M+Na]
Calculated 601.3716, Found 601.3717
(4) Molecular weight: 578
(5) Ultraviolet absorption spectrum (in methanol) : as shown in Fig. 5, specific absorption maximum λmax at 306 nm, 319 nm, 335 nm and 352 nm
(6) Infrared absorption spectrum (KBr Tablet) : as shown in Fig. 6, specific maximum absorption λmax at 3390, 2935, 1727, 1008 cm⁻¹.
(7) Specific rotation: [α]_{D}²³= -15.9° (c= 0.1, methanol)
(8) Solubility in solvent: soluble in dimethyl sulfoxide (DMSO) and methanol, slightly soluble in ethyl acetate and chloroform, and insoluble in hexane and water.
(9) Grouping for acidic, neutral and basic: Neutral substance.
(10) ¹H-proton nuclear magnetic resonance spectrum (in deuteriochloroform) measured by using Varian NMR 400 MHz: Chemical shift of hydrogen (ppm) is shown in Table 3.
(11) ¹³C-nuclear magnetic resonance spectrum (in deuteriochloroform) measured by using Varian NMR 100 MHz: Chemical shift of carbon (ppm) is shown in Table 3.

**Table 3**

| ¹³C | ¹H |
|---|---|
| 174.2 | |
| 45.9 | 2.28 (1H, m) |
| 71.8 | 3.72 (1H, br, m) |
| 41.6 | 1.20 (2H, m) |
| 71.4 | 3.49 (1H ,br, m) |
| 38.8 | 1.20 (2H, m) |
| 22.5 | 1.70 (2H, m) |
| 40.1 | 1.20 (2H, m) |
| 71.4 | 3.49 (1H, br, m) |
| 43.5 | 1.20 (2H, m) |
| 67.7 | 3.41 (1H, m) |
| 45.4 | 1.60 (2H, m) |
| 74.5 | 3.59 (1H, m) |
| 136.7 | 5.54 (1H, dd, J=15.20, 8.75) |
| 130.4 | 6.06 (1H, m) |
| 132.9-131.1 | 6.20 (1H, m) |
| 132.9-131.1 | 6.20 (1H, m) |
| 132.9-131.1 | 6.20 (1H, m) |
| 132.9-131.1 | 6.20 (1H, m) |
| 132.9-131.1 | 6.20 (1H, m) |
| 129.7 | 6.06 (1H, m) |
| 136.1 | 5.80 (1H, dd, J=15.01, 6.69) |
| 35.9 | 2.45 (1H, m) |
| 80.1 | 4.57 (1H, dd, J=8.60, 3.84) |
| 13.8 | 1.09 (3H, d, J=6.96) |
| 10.5 | 0.85 (3H, d, J=6.96) |
| 15.6 | 0.97 (3H, d, J=6.78) |
| 28.7 | 1.93 (1H, m) |
| 19.7 | 0.81 (3H, d, J=5.86) |
| 15.4 | 0.82 (3H, d, J=6.60) |

As shown in above, as a result of detailed examination of various physico-chemical properties and spectral data of K99-5278C substance, K99-5278C substance was determined to have the chemical structure as shown in the formula [III].

Biological properties of K99-5278 substance of the present invention are explained in detail hereinbelow.

### (1) Assays of antifungal activities by paper disc method (filter paper disc method)

Antifungal activities of K99-5278 substance were performed using Aspergillus niger KF103 (ATCC 6275), Mucor racemosus KF223 (IFO 4581), Candida albicans KF1 and Saccharomyces cerevisiae KF26. Aspergillus niger KF103 (ATCC 6275) and Candida albicans KF1 were inoculated at 0.2%, and Mucor racemosus KF223 (IFO 4581) and Saccharomyces cerevisiae KF26 were inoculated at 0.3% in GY agar medium (glucose 1.0%, yeast extract 0.5%, agar 0.8%, pH 6.0). Activities were evaluated by paper disc method (thickness: 6 mm: Advantec Inc., Japan), and a diameter of the inhibition ring was measured after incubating at 27°C for 24 hours. Results were as follows.

K99-5278A substance 10 µg exhibited inhibition zone 18 mm for Aspergillus niger KF103 (ATCC 6275), 9 mm for Mucor racemosus KF223 (IFO 4581), 9 mm for Candida albicans and 12 mm for Saccharomyces cerevisiae KF26.

K99-5278B substance 10 µg exhibited inhibition zone 16 mm for Aspergillus niger KF103 (ATCC 6275), 9 mm for Mucor racemosus KF223 (IFO 4581), 7 mm for Candida albicans and 12 mm for Saccharomyces cerevisiae KF26.

K99-5278C substance 10 µg exhibited inhibition zone 15 mm for Aspergillus niger KF103 (ATCC 6275), 8 mm for Mucor racemosus KF223 (IFO 4581), 8 mm for Candida albicans and 9 mm for Saccharomyces cerevisiae KF26.

### (2) Assays of antifungal activities by liquid culture method

Antifungal activities of K99-5278A substance, K99-5278B substance and K99-5278C substance by the liquid medium were performed as follows.
1) Saccharomyces cerevisiae KF26 was used as a test organism. Samples dissolved in DMSO were added in the 96 well microplate and the solvent was distilled off in vacuo. Suspension, in which Saccharomyces cerevisiae KF26 0.3% was inoculated in a medium (glucose 1.0% and yeast extract 0.5%, pH 6.0), 200 µl/well was added and incubated at 27 °C for 24 hours. After the incubation, turbidity was measured by using absorption spectrometer with wavelength at 550 nm. Activity was determined by setting the value without drug addition as 100%, and the concentration shown by 50% turbidity was set as IC₅₀.
   As a result, IC₅₀ for growth of the test organism in K99-5278A was 7.2 µg/ml; IC₅₀ for growth of the test organism in K99-5278B was 11.8 µg/ml; and IC₅₀ for growth of the test organism in K99-5278C was 16.6 µg/ml.
2) Aspergillus niger KF103 was used as a test organism. Samples dissolved in DMSO were added in the 96 well microplate and the solvent was distilled off in vacuo. Suspension, in which Aspergillus niger KF103 0.3% was inoculated in a medium (glucose 1.0% and yeast extract 0.5%, pH 6.0), 200 µl/well was added and incubated at 27°C for 48 hours. After the incubation, turbidity was measured by using absorption spectrometer with wavelength at 550 nm. Activity was determined by setting the value without drug addition as 100%, and the concentration shown by 50% turbidity was set as IC₅₀.
   As a result, IC₅₀ for growth of the test organism in K99-5278A was 0.32 µg/ml; IC₅₀ for growth of the test organism in K99-5278B was 0.53 µg/ml; and IC₅₀ for growth of the test organism in K99-5278C was 0.75 µg/ml.
3) Mucor racemosus KF223 was used as a test organism. Samples dissolved in DMSO were added in the 96 well microplate and the solvent was distilled off in vacuo. Suspension, in which Mucor racemosus KF223 0.3% was inoculated in a medium (glucose 1.0% and yeast extract 0.5%, pH 6.0), 200 µl/well was added and incubated at 27°C for 24 hours. After the incubation, turbidity was measured by using absorption spectrometer with wavelength at 550 nm. Activity was determined by setting the value without drug addition as 100%, and the concentration shown by 50% turbidity was set as IC₅₀.
   As a result, IC₅₀ for growth of the test organism in K99-5278A was 15 µg/ml; IC₅₀ for growth of the test organism in K99-5278B was 25 µg/ml; and IC₅₀ for growth of the test organism in K99-5278C was 35 µg/ml.
4) Candida albicans KFI was used as a test organism. Samples dissolved in DMSO were added in the 96 well microplate and the solvent was distilled off in vacuo. Suspension, in which Candida albicans KF1 0.3% was inoculated in a medium (glucose 1.0% and yeast extract 0.5%, pH 6.0), 200 µl/well was added and incubated at 27°C for 24 hours. After the incubation, turbidity was measured by using absorption spectrometer with wavelength at 550 nm. Activity was determined by setting the value without drug addition as 100%, and the concentration shown by 50% turbidity was set as IC₅₀.

As a result, IC₅₀ for growth of the test organism in K99-5278A was 61 µg/ml; IC₅₀ for growth of the test organism in K99-5278B was 100 µg/ml; and IC₅₀ for growth of the test organism in K99-5278C was 140 µg/ml.

As described in detail, K99-5278 substance of the present invention can be expected as novel antifungal agent effective for fungi and having novel chemical structure as obvious from the biological properties.

### Brief explanation of drawings

Fig. 1 shows ultraviolet absorption spectrum of K99-5278A substance (in methanol).
Fig. 2 shows infrared absorption spectrum of K99-5278A substance (KBr tablet).
Fig. 3 shows ultraviolet absorption spectrum of K99-5278B substance (in methanol).
Fig. 4 shows infrared absorption spectrum of K99-5278B substance (KBr tablet).
Fig. 5 shows ultraviolet absorption spectrum of K99-5278C substance (in methanol).
Fig. 6 shows infrared absorption spectrum of K99-5278C substance (KBr tablet).

### Best mode for carrying out the invention

The present invention will be explained by illustrating example, but the present invention is not limited within the example. Example

A medium containing glucose 0.1%, starch 2.4%, peptone 0.3%, meat extract 0.3% and CaCO₃ 0.4% (adjusted to pH 7.0), each 10 ml, was added in 500 ml test tubes, cotton sealed and steam sterilized. Streptomyces sp. K99-5278 FERM BP-8198 grown on Seino agar (starch 1%, N-Z amine 0.3%, yeast extract 0.1%, meat extract 0.1%, CaCO₃ 0.3% and agar 1.2%) was aseptically inoculated into the medium and shake cultured at 27°C for 4 days to obtain seed culture liquid.

A medium containing glycerol 1.5%, Marutoku defatted germ (Nisshin Seifun K.K., Japan) 1.0% and CaCO₃ 0.3% (adjusted to pH 7.0), each 100 ml, was added in 500 ml Erlenmeyer flask, 150 flasks, cotton sealed and steam sterilized. The seed culture hereinabove 1 ml was aseptically inoculated into the medium and shake cultured at 27°C for 5 days. Acetone, each 100 ml, was added to the obtained cultured liquid, well shaken and concentrated in vacuo. The residue was treated for extraction with ethyl acetate and concentrated in vacuo to obtain a crude substance 2.4 g.

The crude substance 2.4 g was dissolved in chloroform and was subjected to silica gel column chromatography (100 g, 70 - 230 mm, Merck Inc., the U.S.). A stepwise elution, each 500 ml, with chloroform, and the mixture of chloroform : methanol, 100 : 1, 50 : 1, 10 : 1, 5 : 1 and 1 : 1, respectively. The active component 401 mg was obtained in the fraction of the mixture of chloroform : methanol, 5 : 1. This was further treated with silica gel column chromatography (30 g, 230 - 400 mm, Merck Inc., the U.S.) by elution with the mixture of chloroform : methanol, 5 : 1, to obtain the active component in the fraction Nos. 19 - 27.

The active component was further treated for isolation and purification by HPLC, SSC-5410 (Senshu Kagaku Co., Japan) using column, PEGASIL ODS (ODS resin, 4.6 × 250 mm, Senshu Kagaku Co., Japan). Elution was performed by 50% aqueous acetonitrile, and detection was performed at UV 320 nm, flow rate 1.0 ml/min. As a result, K99-5278A substance 12.8 mg, K99-5278B substance 22.4 mg and K99-5278C substance 8.9 mg were isolated.

### Industrial applicability

As explained hereinabove in detail, K99-5278A substance, K99-5278B substance and K99-5278C substance of the present invention, which were obtained by culturing Streptomyces sp. K99-5278 belonging to genus Streptomyces and having ability to produce K99-5278 substance, accumulating K99-5278 substance in the medium and isolating from the cultured mass, can be expected as the effective pharmaceuticals for mycosis including deep-seated mycosis.

## Claims

1. Novel K99-5278 substance comprising K99-5278A substance which is a compound represented by the formula [I]; 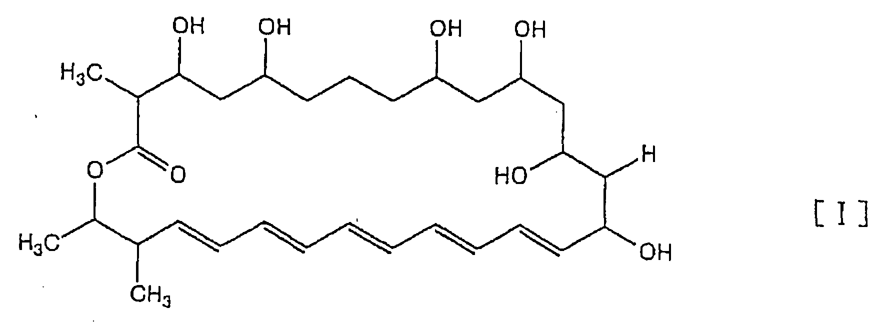 K99-5278B substance which is a compound represented by the formula [II]; 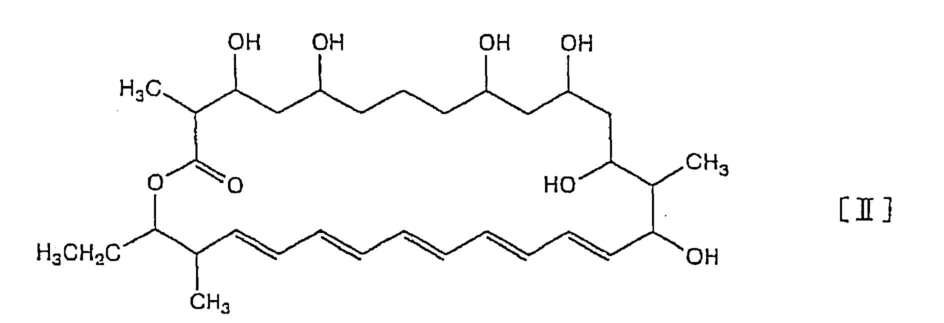 and
K99-5278C substance which is a compound represented by the formula [III];

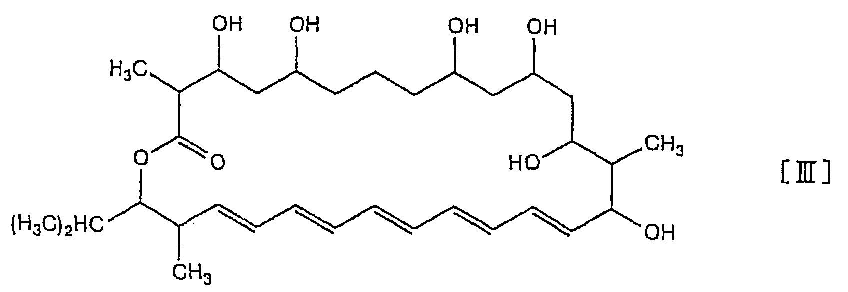

2. A process for production of novel K99-5278 substance comprising culturing a microorganism belonging to genus Streptomyces and having ability to produce K99-5278 substance in a medium, accumulating K99-5278 substance in the cultured medium and isolating K99-5278 substance from the cultured mass.

3. The process for production of novel K99-5278 substance according to claim 2 wherein the microorganism having ability to produce K99-5278 substance is Streptomyces sp. K99-5278 FERM BP-8198 or mutant thereof.

4. A microorganism belonging to genus Streptomyces and having ability to produce substance claimed in claim 1.

5. The microorganism according to claim 4 wherein the microorganism having ability to produce K99-5278 substance is Streptomyces sp. K99-5278 FERM BP-8198 or mutant thereof.

6. The novel K99-5278 substance according to claim 1 wherein K99-5278A substance has antifungal activity against Aspergillus niger KF103, Mucor racemosus KF223, Candida albicans KF1 and Saccharomyces cerevisiae KF26.

7. The novel K99-5278 substance according to claim 1 wherein K99-5278B substance has antifungal activity against Aspergillus niger KF103, Mucor racemosus KF223, Candida albicans KF1 and Saccharomyces cerevisiae KF26.

8. The novel K99-5278 substance according to claim 1 wherein K99-5278C substance has antifungal activity against Aspergillus niger KF103, Mucor racemosus KF223, Candida albicans KF1 and Saccharomyces cerevisiae KF26.
